# EUROPEAN PATENT SPECIFICATION

(11) **EP 1 583 559 B1**
(45) Date of publication and mention of the grant of the patent: **07.04.2010**
(21) Application number: 03810888.2
(22) Date of filing: 23.12.2003
(51) Int. Cl.: A61K 45/06, A61K 33/06, A61K 33/24, A61K 33/26, A61K 33/30, A61K 33/32, A61K 33/34, A61K 31/77, A61K 31/717, A61K 31/28, A61K 31/19, A61K 9/12, A61P 11/00

(54) **COMPOSITIONS FOR PREVENTION AND TREATMENT OF COLD AND INFLUENZA-LIKE SYMPTOMS COMPRISING SELECT MUCOADHESIVE POLYMERS**
ZUSAMMENSETZUNGEN ZUR VORBEUGUNG UND BEHANDLUNG VON ERKÄLTUNG UND INFLUENZA-ÄHNLICHEN ERSCHEINUNGEN ENTHALTEND AUSGEWÄHLTE MUKADHÄSIVE POLYMERE
COMPOSITIONS POUR LA PREVENTION ET POUR LE TRAITEMENT DE RHUMES ET DE SYMPTOMES DE TYPE GRIPPE, COMPRENANT DES POLYMERES MUCOADHESIFS SELECTIONNES

(30) Priority: 13.01.2003 US 341156
(43) Date of publication of application: 12.10.2005
(73) Proprietor: THE PROCTER & GAMBLE COMPANY, Cincinnati, Ohio 45202 (US)
(72) Inventor: KHANOLKAR, Jayant Ekanth, Surbiton, Surrey KT5 8RD (GB); DE LA HARPE, Shane Michael, Liskeard, Cornwall PL14 4BP (GB); MULLET, Benoit Maurice, 28000 Chartres (FR); RENNIE, Paul John, Godalming, Surrey GU7 2HA (GB); WILLIAMS, Philip, David, Egham, Surrey TW20 9RD (GB)
(74) Representative: Phillipson, Ross Peter
(86) International application number: PCT/US2003/041106
(87) International publication number: WO 2004/064867

(56) References cited:
- WO-A-01/28556
- WO-A-04/050059
- US-A- 5 989 535
- US-A- 6 143 329

## Description

### FIELD OF THE INVENTION

The present invention is directed to respiratory tract compositions for the prevention and treatment of cold and influenza-like symptoms due to respiratory tract viral infections, wherein these compositions are effective in both preventing the onset of the symptoms of colds and influenza or significantly mitigating them if an individual is already afflicted with such symptoms. In particular, the present invention is directed to respiratory tract compositions, particularly nasal compositions, comprising select mucoadhesive polymers that provide for improved adherence of the compositions to areas of the respiratory tract for the prevention and treatment of cold and influenza-like symptoms without causing perceived irritation to the areas of the respiratory tract.

### BACKGROUND OF THE INVENTION

It is known that many different viruses and viral strains bring on symptoms associated with respiratory viral infections. The common cold is a complex syndrome caused by over 200 antigenically different viruses found in five virus families. These families include rhinovirus, myxovirus, paramyxovirus, respiratory syncytial virus, adenovirus and coronavirus. The most important group is rhinovirus, Gwaltney J.M., Common Cold, pp 489-493, Mandell G.L., Douglas, R.G. Jr., Bennett, J.E., Principles and Practice of Infectious Diseases, 3rd ed., Churchill Livingstone, New York, 1990. Pinpointing the specific cause of the illness is difficult and not practical since there are also a number of predisposing factors whose contribution to the manifestation of symptoms is not fully understood. Such include, but are not limited to, physical fatigue, psychological stress, and overall physical healthiness.

Regardless of the virus and associated factors leading to the onset of cold and influenza symptoms, a number of remedies to alleviate the symptoms of the common cold have been suggested. The cough/cold products that are currently marketed typically contain one or more of the following actives: nasal decongestants such as pseudoephedrine, oxymetazoline, antihistamines such as doxylamine, antitussives such as dextromethorphan, expectorants such as guaifenesin and anti-pyretics such as acetaminophen. In an attempt to improve existing cold remedies, experts in the field have suggested several alternative pharmacotherapies and subsequently conducted cold trials to test their efficacy. Examples of these therapies include: the use of interferon-α₂, Douglas et al., Prophylactic Efficacy of Intranasal Alpha2- Interferon Against Rhinovirus Infection in the Family Setting, The New England Journal of Medicine, 314, pp. 65-70, 1986; bradykinin antagonist, Higgins et al., A Study of the Efficacy of the Brandykinin Antagonist, NPC567, in Rhinovirus Infections in Human Volunteers, Antiviral Research vol. 14, pp. 339-344, 1990; glucocorticoid, Farr et al., A Randomized Controlled Trial of Glucocorticoid Prophylaxis Against Experimental Rhinovirus Infection, Journal of Infectious Diseases, vol. 162, pp. 1173-1177, 1990; nedocromil, Barrow et al., The Effect of Intranasal Nedocromil Sodium on Viral Upper Respiratory Tract Infections in Human Volunteers, Clinical and Experimental Allergy, vol. 20, pp. 45-51, 1990; a combination of interferon-α₂, ipratropium and naproxen, Cwaltney, Combined Antiviral and Antimediator Treatment of Rhinovirus Colds, The Journal of Infectious Disease vol. 166, pp. 776-782, 1992; zinc salts, Potter et al., DIAS Rounds. Zinc Lozenges for Treatment of Common Colds, The Annals of Pharmacotherapy, vol, 27, pp. 589-592, 1993.

A number of patents have also been issued disclosing compositions for prevention and treatment of the common cold and their methods of use. A sample of such patents includes: US Patents 5,240,694; 5,422,097; and 5,492,689; all to Gwaltney, disclosing treatment using combinations of anti-viral and anti-inflammatory compounds; US Patents Re 33,465 and 5,409,905; both to Eby disclosing treatment using zinc salts; US. Pat. 5,626,831; to Van Moerkerken disclosing treatments using orally administered aminocarboxylic acid compounds; U.S. Patents 4,619,934 and 4,552,899, both to Sunshine, disclosing treatment of cough and colds using compositions comprising non-steroidal anti-inflammatory drugs such as NSAIDS with antihistaminically effective materials such as chlorpheniramine, WO 01/28 556 discloses respiratory tract compositions comprising certain mucoadhesive polymers.

Treatment for influenza includes vaccination and use of specific antiviral drugs. These have been reviewed by A. Elliot and J. Ellis, 2000, Pharmaceutical Journal, 265, 446-451. Amantidine and Rimantidine have been used for treating influenza infections. They target the M2 protein of influenza virus and interfere with release of viral genetic material into the infected cell, thus preventing viral replication. A number of side effects have been reported including neurological and gastro-intestinal complaints, Belshe, R.B., Smith, M.H., Hall, CB, Betts, Hay, R.J., Genetic basis of Resistance to Rimantidine Merging During Treatment of Influenza Virus Infection, Journ. of Virology, 1988, 62, 1508-12; Hay, A.J., The Action of Adamantanamines Against Influenza A Viruses: Inhibition of the M2 Ion Channel. Protein, Semin Virol., 1992, 3, 21-30.

Another approach to influenza treatment has been to inhibit the neuraminidase enzyme molecule on the virus, important to the virus' replication and infectivity. One such treatment drug is Zanamivir, developed by Glaxo Wellcome, Monto, A.S., Robinson, D.P., Herlocher, M.L., Hinson, J.M., Elliot, M.J., Crisp, A., Zanamivir in the prevention of influenza among healthy adults: A randomized controlled trial. JAMA, 1999, 282, 31-5. Side effects reported were sinusitis, diarrhea, nausea, and adverse lung effects. A second neuraminidase inhibiting drug is Oseltamivir, licensed under the trade name Tamiflu, Treanor, J.J., Hayden, F.G., Vrooman, P.S., Bararush, R. Bettis, R., Riff, D. Efficacy and safety of the oral neuraminidase inhibitor Oseltamivir in treating acute influenza: A randomized controlled study. JAMA, 2000, 283, 1016-24. There is a concern with Amantidine, Rimantidine and Neuraminidase inhibitors that viral resistance may develop, rendering them ineffective, A. Elliot and J. Ellis, 2000, Pharmaceutical Journal, 265, 446-451.

US Patent 4,689,223, issued August 25, 1987, assigned to T&R Chemicals, discloses nasal spray compositions for treating the symptoms of or preventing the common cold, wherein the compositions comprise sulphites or bisulphites having low, but, no specific pH is disclosed. EP046409, published February 24, 1982, to Walliczek, discloses processes for the preparation of solution of cuprous complexes for therapeutic treatment of human or animal body; incorporated herein by reference. One disclosed process includes the preparation of cuprous complex with ascorbic acid or non toxic ascorbate having a pH from 4-6. The complex may be used to make solutions for topically treating fungal, inflammatory or viral complaints. US Patent 6,080,783, issued June 27, 2000, assigned to Gum Tech International, Inc., discloses viscous gels for delivering minor effective homeopathic amount of zinc or another metal to the nasal membrane. The compositions maintain ionic zinc in direct contact with the nasal membrane and are believed to provide for the rapid delivery of zinc into the nasal membrane and ultimately into the bloodstream for treating colds. Other disclosures of nasal compositions comprising viscous gels or other viscosity building polymeric materials include U.S. Patent 4,891,226; WO 91/10434; U.S. Patent 4,478,822; U.S Patent 5,599,534; U.S. Patent 5,215,739; U.S. Published Application 2002/0032231; U.S. Patent 6,365,624; U.S. Patent 5,158,761; U.S. Patent 5,897,858; and EP 1108422.

A known characteristic of rhinoviruses is that they lose infectivity under acidic conditions. Even pH values of 5.0 are known to reduce Rhinovirus infectivity, Hughes, J.H., Acid Lability of Rhinovirus Type 14: Effect of pH, Time and Temperature, Proc. Soc. Exp. Biol. Med., 1993, 144, 555-60. EP310317, published April 5, 1989 to Bordt et al., assigned to Beecham, discloses a method for inactivating viruses and bacteria with pharmaceutical compositions including vaccines prepared by inactivating viruses or bacteria with ascorbic acid or its salts in the presence of oxygen and heavy metal ions. US Patents 4,767,788, Diana, issued August 30, 1988, assigned to Sterling Drug Inc., discloses processes for destroying viruses with glutaric acid including rhinovirus in the nasal mucosa.

Despite the abundance of compositions and preventative treatments known in the art, there remains a need to provide a consistent and effective method for prevention and treatment of cold and influenza symptoms. There also remains a need to provide compositions, particularly nasal compositions, that are highly effective in the prevention and treatment of cold and influenza symptoms, wherein the compositions comprise select mucoadhesive polymers that provide for the improved prevention and treatment of these cold and influenza symptoms.

### SUMMARY OF THE INVENTION

The present invention is directed to respiratory tract compositions and uses of such compositions according to the claims. The respiratory tract compositions of the present invention are preferably applied to the areas of the respiratory tract such as the nasal cavity to provide for a surface pH of the nasal cavity tissue of from pH 3.0 to 5.5. These compositions create an environment hostile to viral infections with or without a combination of active ingredients such as a combination of pyroglutamic acid and an organic acid having a pKa value from 3.0 to 5.5.

Preferably, the present invention is directed to nasal compositions, particularly nasal sprays, that have a pH of less than 4.5 and comprise combinations of materials such as metal compounds and organic acids in combination with select mucoadhesive polymers and that further comprise pH adjusting agents wherein the composition has a viscosity of from 1 mPa.s (cps) to 2000 mPa.s (cps).

It has been found that upon application to the nasal tissues, the respiratory tract compositions of the present invention create an environment hostile to viruses. Such an environment deters viruses from infecting the nasal cavity which is subsistent to respiratory tract viral infections. The respiratory tract compositions of the present invention are also suitable for treating already infected subjects in order to mitigate cold and influenza-like symptoms, as well as being suitable for method applications of reducing or eliminating the possibility of acquisition of such viruses when confronted with a high-risk public environment including schools and office buildings.

As previously stated, the respiratory tract compositions of the present invention are especially effective when administered in the preferred embodiment of a nasal composition, particularly a nasal spray, comprising the preferred combination of metal compound, organic acid, and select mucoadhesive polymer.

### DETAILED DESCRIPTION OF THE INVENTION

The compositions of the present invention are respiratory tract compositions that are effective in the prevention and treatment of cold and influenza-like symptoms. These compositions are especially effective in the prevention and treatment of cold and influenza like symptoms when the compositions are administered in the form of nasal compositions. The respiratory tract compositions of the present invention are typically administered using a pharmaceutically acceptable vehicle sometimes referred to as carrier systems.

The term "respiratory tract compositions" as used herein refers to compositions in a form that is directly deliverable to the airway passages from the nose and mouth. These compositions include droppers, pump sprayers, pressurized sprayers, atomizers, air inhalation devices and other packaging and equipment known or yet to be developed.

The respiratory tract compositions are administered to prevent and treat "cold and influenza-like symptoms". As used herein "cold and influenza-like symptoms" refer to symptoms typically associated with respiratory tract viral infections. These symptoms include nasal congestion, chest congestion, sneezing, rhinorrhea, fatigue or malaise, coughing, fever, chills, body ache, sore throat, headache, and other known cold and influenza-like symptoms.

The term "respiratory viruses" as used herein refers to those viruses that are causal agents of cold and influenza-like symptoms. These viruses include Rhinovirus, Myxovirus (Influenza virus), Paramyxovirus (Parainfluenza virus), Respiratory Syncytial virus, Adenovirus and Coronavirus.

The term "pharmaceutically acceptable vehicle" refers to any solid, liquid or gas combined with components of the respiratory tract compositions of the present invention to deliver the components to the respiratory tract of the user. These vehicles are generally regarded as safe for use in humans, and are also known as carrier systems.

The respiratory tract compositions of the present invention can comprise, consist of, or consist essentially of the elements and limitations of the invention described herein, as well as any of the additional or optional ingredients, components, or limitations described herein.

All percentages, parts and ratios are by weight of the respiratory tract compositions, unless otherwise specified. All such weights as they pertain to listed ingredients are based on the specific ingredient level and, therefore, do not include carriers or by-products that may be included in commercially available materials, unless otherwise specified.

### Pyroglutamic Acid

The respiratory tract compositions of the present invention can comprise pyroglutamic acid in combination with an organic acid having a pKa value from 3.0 to 5.5, wherein the combination of said pyroglutamic and organic acids have been shown to provide a surface pH of the nasal cavity tissue of from pH 3.0 to 5.5. The respiratory tract compositions of the present invention comprise the pyroglutamic acid typically at concentrations ranging from 0.01% to 20%, preferably from 0.1% to 10%, more preferably from 0.25% to 8%, most preferably from 0.1% to 5%, by weight of the composition.

The respiratory tract compositions of the present invention are preferably administered in the form of nasal compositions that comprise a safe and effective amount of pyroglutamic acid (PCA) or any other active component included in the compositions, In this context, the term "safe and effective amount" refers to an amount with provides a therapeutic benefit with minimal or no adverse reactions.

The pyroglutamic acid suitable for use in the respiratory tract compositions of the present invention includes those pyroglutamic acid compounds collectively referred to as stereoisomers and tautomers of pyroglutamic acid. Pyroglutamic acid, which is also referred to as pyrrolidone carboxylic acid has two stereoisomers (D and L) and each are preferred for use herein. Pharmaceutically acceptable salts of pyroglutamic acid are also suitable for use herein.

The D stereoisomer of pyroglutamic acid is also know by the following names: D-Proline, 5-oxo-(+)-2-Pyrrolidone-5-carboxylic acid, (+)-Pyroglutamic acid, (R)-2-Pyrrolidone-5-carboxylic acid, 5-Oxo-A-proline, D-2-Pyrrolidone-5-carboxylic acid, D-Pyroglutamic acid, D-Pyrrolidinonecarboxylic acid, and D-Pyrrolidonecarboxylic acid.

The L stereoisomer of pyroglutamic acid is also known by the following names: L-Proline, 5-oxo-(-)-2-Pyrrolidone-5-carboxylic acid, (-)-Pyroglutamic acid, (5S)-2-Oxopyrrolidine-5-carboxylic acid, (S)-(-)-2-Pyrrolidone-5-carboxylic acid, (S)-2-Pyrrolidone-5-carboxylic acid, (S)-5-Oxo-2-pyrrolidinecarboxylic acid, (S)-Pyroglutamic acid, 2-L-Pyrrolidone-5-carboxylic acid, 2-Pyrrolidinone-5-carboxylic acid, 5-Carboxy-2-pyrrolidinone, 5-Oxo-L-proline, 5-Oxoproline, 5-Pyrrolidinone-2-carboxylic acid, Glutimic acid, Glutiminic acid, L-2-Pyrrolidone-5-carboxylic acid, L-5-Carboxy-2-pyrrolidinone, L-5-Oxo-2-pyrrolidinecarboxylic acid, L-5-Oxoproline, L-Glutamic acid, gamma-lactam, L-Glutimic acid, L-Gluriminic acid, L-Pyroglutamic acid, L-Pyrrolidinonecarboxylic acid, L-Pyrrolidonecarboxylic acid, Oxoprolinc, PCA, Pidolic acid, Pyroglutamic acid, Pyrrolidinonecarboxylic acid, Pyrrolidone-5-carboxylic acid, and Pyrrolidonecarboxylic acid.

The DL form of pyroglutamic acid (a mixture of the D and L stereoisomers) is known by the following names: DL-Proline, 5-oxo-(.-+-.)-2-Pyrrolidone-5-carboxylic acid, (.+-.)-Pyroglutamic acid, 5-Oxo-DL-proline, DL-2-Pyrrolidinone-5-carboxylic acid, DL-2-Pyrrolidone-5-carboxylic acid, DL-Pyroglutamate, DL-Pyroglutamic acid, DL-Pyrrolidonecarboxylic acid, and Oxoproline. The DL form is also commercially available from Ajinomoto under the tradenames Ajidew A 100 and Ajidew N 50 (Na-PCA).

Some of the above-listed stereoisomers are commercially available from UCIB, France via Barnet Products Corp., New Jersey. Such compounds are sold under trade names like Cuivridone (Cu-PCA) and L-FER Pidolate (Fe-PCA.), and Pidolidone.

### Organic Acid

The respiratory tract compositions of the present invention comprise an organic acid that is suitable for use in combination with the pyroglutamic acid described hereinabove. It is believed that when the respiratory tract compositions comprise a combination of organic acid and pyroglutamic acid, a composition is created that is hostile to viruses known to contribute to cold and influenza-like symptoms.

The organic acid suitable for use herein can be included in the respiratory tract compositions as an individual organic acid or as a combination of organic acids, provided that the total organic acid concentration ranges from 0.01% to 10%, preferably from 0.05% to 5%, more preferably from 0.10% to 2.5%, by weight of the composition. The organic acid has a dissociation constant (pKa) of from 3.0 to 5.5. It has been shown that when the organic acid is combined with PCA, the respiratory tract compositions have an increased buffering capacity, providing a surface pH of the tissue treated in the nasal cavities or turbinates of from 3.0 to 5.5.

Examples of an organic acid suitable for use herein include ascorbic acid, monocarboxylic acids, dicarboxylic acids, tricarboxylic acids, and mixtures thereof. Specific examples of suitable monocarboxylic, dicarboxylic, or tricarboxylic acids include salicylic, fumaric, benzoic, glutaric, lactic, citric, malonic, acetic, glycolic, malic, adipic, succinic, aspartic, phthalic, tartaric, glutamic, gluconic, and mixtures thereof. It has been found that these organic acids can be incorporated into the respiratory tract compositions of the present invention at the defined concentrations to create a hostile environment for viruses without significantly irritating specific areas of the respiratory tract such as the nasal tissues, notwithstanding that organic acids have been known to be potential irritants to respiratory tract tissues.

Although the organic acid is effective when used in combination with pyroglutamic acid, it has been found that combinations of the organic acid and metal compounds are also effective in the prevention and treatment of cold and influenza-like symptoms. Combinations of the organic acid and metal compound can be included in the respiratory tract compositions with or without the addition of pyroglutamic acid to provide a therapeutic benefit in the prevention and treatment of cold and influenza-like symptoms

### Metal Compound

The respiratory tract compositions of the present invention comprise a safe and effective amount of a metal compound, preferably a metal compound in combination with the organic acid described hereinabove. The metal compound is commonly referred to' as a "metal salt", wherein metal ion substituents such as iron, silver, copper, and zinc are believed to be primary components of a metal compound that provide for a hostile environment for cold and influenza viruses.

The concentration of the metal compound in the respiratory tract compositions of the present invention typically ranges from 0.001% to 20% preferably from 0.01% to 10%, more preferably from 0,05% to 5%, most preferably from 0.05% to 2%, by weight of the composition. The metal compound can be included in the respiratory tract compositions as an individual metal compound or as a combination of metal compounds, wherein the total concentration of metal compound typically ranges from 0.001% to 20% by weight of the composition.

As previously stated a preferred embodiment of the respiratory tract compositions of the present invention is wherein the compositions comprise a combination of organic acid and metal compound. When the respiratory tract compositions comprise this combination of ingredients, the organic acid is included at concentrations ranging from 0.01% to 5%, preferably from 0.1% to 2.5% by weight of the composition, wherein the metal compound is included at concentrations ranging from 0.05% to 10%, preferably from 0.1 % to 2.5% by weight of the composition.

Alternatively, the respiratory tract compositions of the present invention can comprise the metal compound and organic acid in combination with pyroglutamic acid. Without being limited by theory, it is believed that when the respiratory tract compositions of the present invention include the metal compound, the organic acid, and pyroglutamic acid, the metal compound and pyroglutamic acid can form a metal-acid complex that can provide for a synergistic immediate and residual anti-viral effect, The metal compound can be combined with pyroglutamic acid to form a metal-acid complex prior to incorporation of the metal-acid complex into the respiratory tract compositions of the present invention. If the metal-acid complex is formed prior to inclusion in the respiratory tract compositions herein, the metal-acid complex is included at concentrations ranging from 0.001% to 20%, preferably from 0.01% to 10%, more preferably from 0.1% to 5%, by weight of the composition. It is also contemplated that the respiratory tract compositions of the present invention can comprise the combination of metal compound and pyroglutamic acid without the inclusion of an organic acid.

The metal compound comprises a metal ion selected from the group consisting of manganese (Mn), silver (Ag), zinc (Zn), tin (Sn), iron (Fe), copper (Cu), aluminum (Al), nickel (Ni), cobalt (Co), or mixtures thereof. Preferred metal compounds include those metal compounds which contain Cu, Fe, or Zn metal ions, or combinations thereof.

Examples of a metal compound suitable for use herein include the metal compounds referred to as salicylates, furnarates, benzoates, glutarates, lactates, citrates, malonates, acetates, glycolates, thiosalicylates, adipates, succinates, gluconates, aspartates, glycinates, tartarates, malates, maleates, ascorbates, chlorides, sulphates, nitrates, phosphates, fluorides, iodides, pidolates, and mixtures thereof. The acetates, ascorbates, chlorides, benzoates, citrates, gluconates, glutarates, lactates malates, malonates, salicylates, succinates, sulphates, or mixtures thereof are preferred metal compounds.

Specific examples of a metal compound suitable for use herein include zinc acetate, zinc chloride, zinc ascorbate, zinc gluconate, zinc pidolate, zinc succinate, zinc sulphate, zinc chloride, or mixtures thereof. Zinc acetate is the most preferred metal compound.

When the respiratory tract compositions of the present invention comprise a metal compound containing zinc ion, it is believed that the zinc ion provides for antiviral properties. Furthermore, it is known that metal ions such as iron, silver, copper, and zinc can provide antiviral properties for the prevention and treatment of cold and influenza-like symptoms. Particularly, zinc and its possible effects on common colds has been extensively documented, The Handbook for Curing the Common Cold, George A. Eby, published 1994, George Eby Research, Texas, USA. The mechanism of its action is thought to be multifactorial. Zinc ions have been shown to be both antiviral and antibacterial. They are believed to inhibit cleavage of rhinovirus polypeptides, preventing replication and formation of infective virions. Zinc ions reduce the ability of rhinoviruses to penetrate cell membranes, partly by lowering expression of intercellular adhesion molecule ICAM. Zinc ions have also been shown to stimulate T-cell lyphocytes, including production of the natural antiviral, interferon-gamma. They stabilize cell plasma membranes, protecting cells from cytotoxic agents, and preventing cell leakage.

### Mucoadhesive Polymer

The respiratory tract compositions of the present invention comprise a mucoadhesive polymer that provides for improved retention of the compositions in areas of the respiratory tract such as the nasal cavity to result in improved prevention and treatment of cold and influenza-like symptoms without causing nasal irritation. It is known that mucoadhesive polymers can be incorporated into respiratory tract compositions such as nasal compositions to exhibit stimulus responsiveness. By stimulus responsiveness, it is meant that upon contacting the mucosal fluids or tissues, the compositions become sufficiently tacky or viscous to adhere to the tissues and do not quickly erode from the surface.

The mucoadhesive polymers herein impart a change in the viscosity of the respiratory tract compositions upon contact of the compositions with stimulus such as pH, body temperature, change in ionic concentration, and the like. Therefore, mucoadhesive polymers are sometimes commonly referred to as viscosity building polymers. It has been found that the incorporation of a mucoadhesive polymer that provides for a change in viscosity results in reducing and/or eliminating perceived irritation, especially perceived nasal irritation, that can be caused by the acidic nature of the respiratory tract compositions of the present invention.

The mucoadhesive polymers herein provide for the adherence of the respiratory tract compositions to mucosal tissues, particularly nasal mucosal tissues, such that the acidic respiratory tract composition comes into contact with mucosal tissues and fluids to result in a change in viscosity of the compositions in the respiratory tract arca. As a result, the respiratory tract compositions of the present invention are maintained on the mucosal surface for periods longer than typical respiratory tract compositions, thus maintaining a virus-hostile environment for the improved prevention and treatment of cold and influenza-like symptoms. For example, if the respiratory tract compositions of the present invention are administered as a liquid respiratory tract composition, the composition is applied using an atomizing sprayer, and upon spraying into a respiratory tract area such as the nasal cavity the composition quickly forms a polymeric film, preferably a polymeric viscous film, that adheres to the nasal tissues. The polymeric film is preferably a thin polymer film, more preferably a thin polymeric viscous film, that is also resistant to erosion upon sneezing, blowing of the nose, or upon mucociliary clearance. The mucoadhesive polymer is also capable of changing the viscosity of the compositions in situ upon application of the respiratory tract compositions to the mucosal tissues and fluids.

The mucoadhesive polymers herein can be included in the respiratory tract compositions of the present invention of a concentration of from 0.01% to 30%, preferably from 0.1% to 20%, more preferably from 1% to 15%, by weight of the composition.

The incorporation of the mucoadhesive polymer into the respiratory tract compositions of the present invention results in a composition that has a viscosity in the range of from about 1 mPa.s (centipoise (cps)) to about 2000 mPa.s (cps) preferably from about 1 mPa.s (cps) to about 1000 mPa.s (cps), more preferably from about 5 mPa.s (cps) to about 500 mPa.s (cps) most preferably from about 5 mPa.s (cps) to about 300 mPa.s (cps). The viscosity of the compositions can be measured by any known or otherwise effective technique employed to determine viscosity. Generally, the viscosity of the respiratory tract compositions of the present invention is determined using known methods such as those described in ASTM D1824-87, ASTM D1084-88, and ASTM D2196-86. The viscometer employed to measure viscosity herein is the Haake Viscometer. The Haake Viscometer utilized is the Rheostress 1 model equipped with probe C35/2T wherein the viscosity measurement is performed over a temperature range of 0°C to 5°C at 50 revolutions per minute (rpm)/second (sec).

The mucoadhesive polymers for use herein are polymeric cellulose derivatives, and thermoreversible polymer.

Specific examples of polymeric cellulose derivatives suitable for use as a preferred mucoadhesive polymer herein include hydroxy alkyl cellulose polymers including hydroxypropyl methylcellulose (HPMC) and hydroxypropyl cellulose (HPC), methyl cellulose polymers, carboxymethyl cellulose (CMC) polymers, salts of carboxymethyl cellulose including sodium salt of carboxymethyl cellulose, or mixtures thereof.

Specific examples of thermoreversible polymers suitable for use as a preferred mucoadhesive polymer herein include poloxamers including those poloxamers sold under the Lutrol F-127 and Lutrol F-68 tradenames, ethylhydroxy ethylcellulose (EHEC), or mixtures thereof.

The mucoadhesive polymer suitable for use herein is more fully described in the Journal Pharmacy Pharmacology 53, pages 3-22, (2001 Edition); the International Journal of Pharmaceutics (1988, 1996 and 1998 Editions); and the Journal Controlled Release 62, pages 101-107, (1999 Edition).

### Pharmaceutically Acceptable Vehicle

The respiratory tract compositions of the present invention are typically administered to respiratory tract areas as formulations comprising a pharmaceutically acceptable vehicle or carrier system. Any pharmaceutically acceptable vehicle in the form of a liquid, solid, or gas is suitable for the delivery of the respiratory tract compositions to prevent and treat cold and influenza-like symptoms.

Depending on the desired form and delivery device to be used, the respiratory tract compositions of the present invention can be combined with pharmaceutically acceptable vehicles such as water, water-miscible solvents including ethanol, propylene glycol, polyethylene glycol, transcutol, glycerol, and other known or otherwise effective water-miscible solvents; liquid aerosol propellants; and mixtures thereof. Preferably these vehicles are isotonic with human plasma.

When the respiratory tract compositions are administered using water as a pharmaceutically acceptable vehicle, the water is preferably purified or de-ionized water and is free of organic impurities. The concentration of water utilized to formulate the respiratory tract compositions into a final product form for delivery to respiratory tract areas ranges from 40% to 99.98%, preferably from 80% to 99-95%, by weight of the final product formulation.

When the respiratory tract compositions of the present invention are administered using a solid pharmaceutically acceptable vehicle, the vehicle may be applied in a powder form. In other words, the respiratory tract compositions of the present invention can be applied as a solid powder containing the essential ingredients and any optional components described herein with or without any known or otherwise effective solidification aids. However, pharmaceutically acceptable solid vehicles can be added to provide aid in processing of the compositions, to aid in the consistency of the compositions, to provide for improved stability, to facilitate handling, for hygroscopicity benefits, and so forth. Pharmaceutically acceptable solid vehicle materials include ingredients such as particulate and powder fillers, for example, a lactose powder. For respiratory tract compositions in the form of nasal compositions that are administered using a solid powder pharmaceutically acceptable vehicle, the particle size of the powder is typically greater than 10 microns, especially when the nasal composition is a nasal inhalant.

### Optional Components

The respiratory tract compositions of the present invention may further comprise one or more optional components known or otherwise effective for use in pharmaceutical compositions, provided that the optional components are physically and chemically compatible with the essential components described hereinabove, or do not otherwise unduly impair product stability, aesthetics, or performance. Optional components suitable for use herein include materials such as pH adjusting agents, chelating agents preservatives, sensates, sweeteners, flavors, volatile oils, mucilages, surfactant spreading aids including polyoxyethylene (20) sorbitan mono-oleate commercially sold as Polysorbate 80, and so forth. The optional components can be included in the respiratory tract compositions at concentrations ranging from 0.001% to 20%, preferably from 0.01 % to 10%, by weight of the composition.

The respiratory tract compositions of the present invention can optionally comprise homeopathic ingredients. A detailed, but not necessarily a complete list, of such homeopathic ingredients is found in The Homeopathic Pharmacopoeia of the United States, 1999 ed., published by The Pharmacopoeia Convention of the American Institute of Homeopath, ©1982, Vol. 1-4. Specific examples of known, homeopathic, or otherwise effective, optional components suitable for use herein are described in more detail hereinbelow.

The compositions of the present invention comprise pH adjusting agents and have a pH of less than 4.5. Therefore, when the compositions are applied to respiratory tract areas such as nasal tissues, the pH of the composition on the nasal tissues remains from about 3.0 to about 5.5, but is not so low as to cause irritation of the nasal tissues. Optional pH adjusting agents include those normally associated with use in nasal compositions including compounds such as sodium bicarbonate, sodium phosphate, sodium hydroxide, ammonium hydroxide, sodium stannate, triethanolamine, sodium citrate, disodium succinate, and mixtures thereof. If present, the optional pH adjusting agents are generally included at concentrations ranging from 0.01% to 5.0% by weight of the composition.

Another specific example of an optional component suitable for use herein include chelating agents which are believed to provide for enhanced antiviral activity, Optional chelating agents useful in the respiratory tract compositions of the present invention include those that chelate transition metal ions such as iron, copper, zinc and other such metals. Not to be bound by theory, it is reasonable to postulate that metal ions, specifically metal cations, play a major role in the formation of oxidizing species. Oxidizing reactions and free radical formation can contribute to cellular damage in inflammatory diseases. The optional chelating agents useful herein are known to dampen oxidation reactions. The optional chelating agents are stable and effective in non-aqueous and aqueous mediums, and at pH ranges between about 3 to about 6. Nonlimiting examples of suitable optional chelating agents include phytic acid, disodium and calcium salts of ethylene diamine tetraacetic acid (EDTA), tetrasodium EDTA, sodium hexametaphosphate (SHMP), di(hydroxyethyl)glycine, 8-hydroxyquinoline, and mixtures thereof. If the respiratory tract compositions of the present invention comprise one or more optional chelating agents, the chelating agents are included at concentrations ranging from 0.001% to 10.00%, preferably from 0.005% to 5.0%, more preferably from 0.01% to 2%, by weight of the composition.

Other specific nonlimiting examples of optional components suitable for use herein include optional preservatives. Preservatives can optionally be included to prevent microbial contamination that can be attributed to dosing devices or the respiratory tract composition applied to the nose. Such optional preservatives include those normally associated with use in nasal compositions including benzalkonium chloride, chlorhexidine gluconate, phenyl ethyl alcohol, phenoxyethanol, benzyl alcohol, sorbic acid, thimerosal, phenylmercuric acetate, and mixtures thereof.

### Method of Manufacture

The respiratory tract compositions of the present invention may be prepared by any known or otherwise effective technique suitable for providing a pharmaceutical composition that provides a therapeutic benefit in the prevention and treatment of cold and influenza-like symptoms. The respiratory tract compositions are preferably formulated to comprise the metal compound, organic acid, and mucoadhesive polymer described herein, wherein these compositions are then manufactured into final product forms of liquids, sprays, powders, inhalants, pumps, drops, and so forth for administration to respiratory areas to prevent and treat symptoms due to respiratory tract viral infections.

When the respiratory tract compositions are administered using a pharmaceutically acceptable vehicle such as a liquid to deliver the compositions in product forms of sprays, pumps, droplets, and the like, the respiratory tract compositions are generally prepared by solubilizing a mucoadhesive polymer in a liquid vehicle such as water. While stirring, pyroglutamic acid if desired, a metal compound, and an organic acid are then added to the polymer solution. Next, a sensate mix is added while the solution is allowed to continue stirring. The sensate mix is typically added as a premix solution that can contain a combination of ingredients such as a combination of ethanol, menthol, peppermint oil, and spearmint oil. The pH of the resultant product should be less than 4.5, however, a pH adjusting agent such as sodium hydroxide and/or disodium succinate can be added to maintain the pH of the resultant product to values less than 4.5. These respiratory tract compositions administered in their liquid final product forms are especially suitable for incorporation into fill dropper vials, wherein the compositions are sprayed into a respiratory tract areas such as the nostrils or turbinates for effective prevention and treatment of cold and influenza-like symptoms. Typically, about 100 microliters of the composition are sprayed into each nostril or turbinate.

When the respiratory tract compositions of the present invention are administered using a pharmaceutically acceptable vehicle such as a powder, the compositions are generally prepared by dry blending pyroglutamic acid if desired, a metal compound, and an organic acid using a V-mixer. A pH adjusting agent such as sodium citrate can be added to the dry blend. The dry blend is then micronized using a fluid energy mill, The resultant micronized dry blend is then dry mixed with a powder filler such as lactose powder. This final powder respiratory tract composition can optionally be coated with a sensate premix using known spray coating techniques. The final powder respiratory tract composition can be filled into a nasal inhalation metering pump to prevent and treat symptoms of the cold and influenza, wherein about 10 milligrams (mgs) of the final powder can be administered to a respiratory tract area such as a nostril or a turbinate.

As stated herein, the respiratory tract compositions of the present invention are suitable for administration in final product forms of liquids, sprays, pumps, inhalants, powders, and so forth. Suitable devices utilized in the administration of these final respiratory tract compositions include those commonly employed or otherwise effective liquid containers, droppers, spray containers including pressurized sprayers, pump containers, inhalation devices, powder containers, atomizers, and so forth.

### EXAMPLES

The following examples further describe and demonstrate embodiments within the scope of the present invention. The examples are given solely for the purpose of illustration and are not to be construed as limitations of the present invention. All exemplified concentrations are weight-weight percents, unless otherwise specified.

Exemplary respiratory tract compositions of the present invention are exemplified in Table Il hereinbelow. These respiratory tract compositions preferably comprise a sensate premix exemplified in Table I hereinbelow. The exemplified sensate premixes of Table I provide for respiratory tract compositions that are aesthetically pleasing in taste, flavor, coolness, smell, and the like.

The respiratory tract compositions exemplified hereinbelow in Table II are typically prepared by solubilizing a mucoadhesive polymer in a liquid vehicle such as water. While stirring, the pyroglutamic acid if desired, the metal compounds, and organic acid are then added to the polymer solution. Next, the sensate mix is added while the solution is allowed to continue stirring. The pH of the resultant solution should be between about 3.0 and about 5.5, however, a pH adjusting agent such as sodium hydroxide and/or disodium succinate can be added to maintain the pH of the resultant solution to values less than 4.5. These final respiratory tract compositions are suitable for incorporation into fill dropper vials, wherein the compositions are sprayed into a respiratory tract areas such as the nostrils or turbinates for effective prevention and treatment of cold and influenza-like symptoms. Typically, about 100 microliters of the composition are sprayed into each nostril or turbinate.

**Table I**

| **Component** | **Sensate Mix A** | **Sensate Mix B** | **Sensate Mix C** |
|---|---|---|---|
| | **(Wt. %)** | **(Wt. %)** | **(Wt. %)** |
| Ethanol | 47.16 | -- | -- |
| Menthol | 29.41 | 11.565 | 8.878 |
| Peppermint Oil | 17.61 | -- | -- |
| Spearmint Oil | 5.82 | -- | -- |
| Phenyl Ethyl Alcohol | -- | 77.495 | 83.281 |
| Camphor | -- | 6.971 | 4.998 |
| Eucalyptol | -- | 3.969 | 2.843 |
| **Total:** | **100** | **100** | **100** |

**Table II**

| **Component** | **Sample 1** | **Sample 2** | **Sample 3** | **Sample 4** | **Sample 5** |
|---|---|---|---|---|---|
| | **(Wt.%)** | **(Wt.%)** | **(Wt. %)** | **(Wt. %)** | **(Wt. %)** |
| Pyroglutamic Acid¹ | 0.35 | 0.70 | 1.00 | 1.35 | -- |
| Succinic Acid² | 1.00 | 0.70 | 0.35 | -- | 1.35 |
| Zinc Acetate Dihydrate³ | 0.12 | 0.012 | 0.12 | 1.20 | 1.20 |
| Polysorbate 80 | 0.05 | 0.05 | 0.05 | 0.10 | 0.10 |
| Carbopol 980⁴ | -- | -- | 1.20 | 0.01 | -- |
| Hydroxypropyl methyl cellulose⁵ | 1.20 | -- | -- | 0.30 | 0.60 |
| Lutrol F-127⁶ | -- | 15.8 | -- | 5.00 | 7.90 |
| Sodiun Sacharrin | -- | 0.025 | 0.025 | 0.025 | 0.01 |
| Sucrolose | 0.025 | -- | -- | -- | 0.01 |
| Phenyl ethyl alcohol | 0.37 | 0.37 | 0.35 | 0.40 | 0.50 |
| Sodium chloride | 0.20 | 0.20 | 0.50 | 0.25 | 0.20 |
| Sensate Mix A | 0.067 | -- | -- | 0.07 | -- |
| Sensate Mix B | -- | 0.49 | 0.45 | -- | 0.50 |
| Sodium hydroxide (30%) | -- | -- | 0.10 | 0.30 | -- |
| Disodium succinate | 1.00 | 0.50 | -- | -- | 2.50 |
| Deionized Water | q.s. 100 | q.s. 100 | q.s. 100 | q.s. 100 | q.s. 100 |

| | | | | | |
|---|---|---|---|---|---|
| Wt. % - weight percent 1 - pyroglutamic acid available from UCIB, France via Barnet Products Corp., New Jersey 2 - succinic acid available from DSM Fine Chemicals, UK 3 - zinc acetate dihydrate available from Verdugt B. V., Belgium 4-Carbopol 980 available from B. F. Goodrich Company, USA. 5 - hydroxypropyl methylcellulose available from Colorcon Ltd, Kent, UK 6 - Lutrol F-127 available from BASF Speciality Chemicals, Mount Oliver, NJ, USA | | | | | |

## Claims

1. A respiratory tract composition comprising:
(a) from 0.001% to 20% by weight of a metal compound comprising a metal ion selected from the group consisting of manganese (Mn), silver (Ag), zinc (Zn), tin (Sn), iron (Fe), copper (Cu), aluminum (Al), nickel (Ni), cobalt (Co), or mixtures thereof;
(b) from 0.01 % to 10% by weight of an organic acid; and
(c) from 0.01% to 30% by weight of a mucoadhesive polymer selected from polymeric cellulose derivatives and thermoreversible polymers;
wherein the composition has a viscosity of from 1 mPa.s (cps) to 2000 mPa.s (cps) at a temperature of from 0°C to 5°C and further comprising pH adjusting agents wherein the pH of the composition is less than 4.5.

2. The respiratory tract composition of Claim 1 wherein the metal compound is a metal salt selected from the group consisting of salicylates, fumarates, benzoates, glutarates, lactates, citrates, malonates, acetates, glycolates, thiosalicylates, adipates, succinates, gluconates, aspartates, glycinates, tartarates, malates, maleates, ascorbates, chlorides, sulphates, nitrates, phosphates, fluorides, iodides, pidolates, or mixtures thereof.

3. The respiratory tract composition of Claim 2 wherein the metal compound is an acetate metal compound.

4. The respiratory tract composition of Claim 3 wherein the acetate metal compound is zinc acetate.

5. The respiratory tract composition according to any one of claims 1 to 4 wherein the organic acid is selected from the group consisting of ascorbic acid, salicylic acid, fumaric acid, benzoic acid, glutaric acid, lactic acid, citric acid, malonic acid, acetic acid, glycolic acid, malic acid, adipic acid, succinic acid, aspartic acid, phthalic acid, tartaric acid, glutamic acid, gluconic acid, or mixtures thereof.

6. The respiratory tract composition according to any one of claims 1 to 5 wherein the mucoadhesive polymer is a polymeric cellulose derivative selected from the group consisting of hydroxypropyl methylcelluloses, hydroxypropyl celluloses, methyl cellulose polymers, carboxymethyl cellulose polymers, salts of carboxymethyl cellulose, or mixtures thereof.

7. The respiratory tract composition according to any one of claims 1 to 5 wherein the mucoadhesive polymer is a thermoreversible polymer selected from the group consisting of poloxamers, ethylhydroxy ethylcelluloses, or mixtures thereof.

8. The respiratory tract composition of Claim 6 wherein the composition has a viscosity of from 20 mPa.s (cps)to 500 mPa.s (cps).

9. The respiratory tract composition of Claim 7 wherein the composition has a viscosity of from 10 mPa.s (cps)to 600 mPa.s (cps).

10. The respiratory tract composition according to any one of claims 1 to 9 wherein the composition further comprises a pH adjusting agent selected from the group consisting of sodium bicarbonate, sodium phosphate, sodium hydroxide, ammonium hydroxide, sodium stannate, triethanolamine, sodium citrate, disodium succinate, or mixtures thereof.

11. The respiratory tract composition according to any one of claims 1 to 10 wherein the composition further comprises from 0.01 % to 20% by weight of pyroglutamic acid.

12. The respiratory tract composition according to any one of claims 1 to 11 wherein the composition is a nasal composition.

13. The respiratory tract composition of Claim 12 wherein the nasal composition is selected from the group consisting of nasal liquids, nasal sprays, nasal inhalants, nasal powders, nasal drops, or mixtures thereof,

14. The respiratory tract composition of Claim 13 wherein the nasal composition is a nasal spray.

15. The respiratory tract composition according to claim 14 wherein the nasal spray comprises from 40% to 99.98% by weight of a pharmaceutically acceptable vehicle selected from the group consisting of water ethanol, propylene glycol, polyethylene glycol, transcutol, glycerol, a liquid aerosol propellant, or mixtures thereof.

16. The use of a composition according to any one of claims 1 to 11 as a medicament.

17. The use of a composition according to any one of claims 1 to 11 in the manufacture of a medicament for the treatment of a cold or influenza.

18. The use according to claim 16 or claim 17 wherein the medicament is in the form of a nasal spray.

## Patentansprüche

1. Atemwegs-Zusammensetzung, umfassend:
(a) zu 0,001 Gew.-% bis 20 Gew.-% eine Metallverbindung, die ein Metallion, ausgewählt aus der Gruppe bestehend aus Mangan (Mn), Silber (Ag), Zink (Zn), Zinn (Sn), Eisen (Fe), Kupfer (Cu), Aluminium (A1), Nickel (Ni), Cobalt (Co) oder deren Mischungen, umfasst;
(b) zu 0,01 Gew.-% bis 10 Gew.-% eine organische Säure; und
(c) zu 0,01 Gew.-% bis 30 Gew.-% ein mukoadhäsives Polymer, ausgewählt aus polymeren Cellulosederivaten und thermoreversiblen Polymeren;
wobei die Zusammensetzung eine Viskosität von 1 mPa.s (cps) bis 2000 mPa.s (cps) bei einer Temperatur von 0 °C bis 5 °C aufweist und ferner pH-Wert-Einstellmittel umfasst, wobei der pH-Wert der Zusammensetzung unter 4,5 liegt.

2. Atemwegs-Zusammensetzung nach Anspruch 1, wobei die Metallverbindung ein Metallsalz ist, das ausgewählt ist aus der Gruppe bestehend aus Salicylaten, Fumaraten, Benzoaten, Glutaraten, Lactaten, Citraten, Malonaten, Acetaten, Glycolaten, Thiosalicylaten, Adipaten, Succinaten, Gluconaten, Aspartaten, Glycinaten, Tartaraten, Malaten, Maleaten, Ascorbaten, Chloriden, Sulphaten, Nitraten, Phosphaten, Fluoriden, Iodiden, Pidolaten oder deren Mischungen.

3. Atemwegs-Zusammensetzung nach Anspruch 2, wobei die Metallverbindung eine Acetatmetallverbindung ist.

4. Atemwegs-Zusammensetzung nach Anspruch 3, wobei die Acetatmetallverbindung Zinkacetat ist.

5. Atemwegs-Zusammensetzung nach einem der Ansprüche 1 bis 4, wobei die organische Säure ausgewählt ist aus der Gruppe bestehend aus Ascorbinsäure, Salicylsäure, Fumarsäure, Benzoesäure, Glutarsäure, Milchsäure, Citronensäure, Malonsäure, Essigsäure, Glycolsäure, Äpfelsäure, Adipinsäure, Bernsteinsäure, Asparaginsäure, Phthalsäure, Weinsäure, Glutaminsäure, Gluconsäure oder deren Mischungen.

6. Atemwegs-Zusammensetzung nach einem der Ansprüche 1 bis 5, wobei das mukoadhäsive Polymer ein polymeres Cellulosederivat ist, das ausgewählt ist aus der Gruppe bestehend aus Hydroxypropylmethylcellulosen, Hydroxypropylcellulosen, Methylcellulose-Polymeren, Carboxymethylcellulose-Polymeren, Salzen von Carboxymethylcellulose oder deren Mischungen.

7. Atemwegs-Zusammensetzung nach einem der Ansprüche 1 bis 5, wobei das mukoadhäsive Polymer ein thermoreversibles Polymer ist, das ausgewählt ist aus der Gruppe bestehend aus Poloxameren, Ethylhydroxyethylcellulosen oder deren Mischungen.

8. Atemwegs-Zusammensetzung nach Anspruch 6, wobei die Zusammensetzung eine Viskosität von 20 mPa.s (cps) bis 500 mPa.s (cps) aufweist.

9. Atemwegs-Zusammensetzung nach Anspruch 7, wobei die Zusammensetzung eine Viskosität von 10 mPa.s (cps) bis 600 mPa.s (cps) aufweist.

10. Atemwegs-Zusammensetzung nach einem der Ansprüche 1 bis 9, wobei die Zusammensetzung ferner ein pH-Wert-Einstellmittel umfasst, das ausgewählt ist aus der Gruppe bestehend aus Natriumbicarbonat, Natriumphosphat, Natriumhydroxid, Ammoniumhydroxid, Natriumstannat, Triethanolamin, Natriumcitrat, Dinatriumsuccinat oder deren Mischungen.

11. Atemwegs-Zusammensetzung nach einem der Ansprüche 1 bis 10, wobei die Zusammensetzung ferner zu 0,01 Gew.-% bis 20 Gew.-% Polyglutaminsäure umfasst.

12. Atemwegs-Zusammensetzung nach einem der Ansprüche 1 bis 11, wobei die Zusammensetzung eine Nasal-Zusammensetzung ist.

13. Atemwegs-Zusammensetzung nach Anspruch 12, wobei die Nasal-Zusammensetzung ausgewählt ist aus der Gruppe bestehend aus Nasal-Flüssigkeiten, Nasensprays, Naseninhalationsmitteln, Nasenpudem, Nasentropfen oder deren Mischungen.

14. Atemwegs-Zusammensetzung nach Anspruch 13, wobei die Nasal-Zusammensetzung ein Nasenspray ist.

15. Atemwegs-Zusammensetzung nach Anspruch 14, wobei das Nasenspray zu 40 Gew.-% bis 99,98 Gew.-% einen pharmazeutisch annehmbaren Träger umfasst, der ausgewählt ist aus der Gruppe bestehend aus Wasser, Ethanol, Propylenglycol, Polyethylenglycol, Transcutol, Glycerol, einem flüssigen Aerosoltreibmittel oder deren Mischungen.

16. Verwendung einer Zusammensetzung nach einem der Ansprüche 1 bis 11 als Medikament.

17. Verwendung einer Zusammensetzung nach einem der Ansprüche 1 bis 11 bei der Herstellung eines Medikaments für die Behandlung einer Erkältung oder Influenza.

18. Verwendung nach Anspruch 16 oder Anspruch 17, wobei das Medikament in Form eines Nasensprays vorliegt.

## Revendications

1. Composition pour le système respiratoire comprenant :
(a) de 0,001 % à 20 % en poids d'un composé métallique comprenant un ion métallique choisi dans le groupe constitué de manganèse (Mn), argent (Ag), zinc (Zn), étain (Sn), fer (Fe), cuivre (Cu), aluminium (Al), nickel (Ni), cobalt (Co), ou leurs mélanges ;
(b) de 0,01 % à 10 % en poids d'un acide organique ; et
(c) de 0,01 % à 30 % en poids d'un polymère muco-adhésif choisi parmi les dérivés de cellulose polymères et les polymères thermoréversibles ;
où la composition a une viscosité allant de 1 mPa.s (cP) à 2000 mPa.s (cP) à une température allant de 0 °C à 5 °C et comprenant en outre des agents d'ajustement du pH, dans laquelle le pH de la composition est inférieur à 4,5.

2. Composition pour le système respiratoire selon la revendication 1, dans laquelle le composé métallique est un sel métallique choisi dans le groupe constitué de salicylates, furnarates, benzoates, glutarates, lactates, citrates, malonates, acétates, glycolates, thiosalicylates, adipates, succinates, gluconates, aspartates, glycinates, tartrates, malates, maléates, ascorbates, chlorures, sulfates, nitrates, phosphates, fluorures, iodures, pidolates, ou leurs mélanges.

3. Composition pour le système respiratoire selon la revendication 2, dans laquelle le composé métallique est un composé métallique acétate.

4. Composition pour le système respiratoire selon la revendication 3, dans laquelle le composé métallique acétate est de l'acétate de zinc.

5. Composition pour le système respiratoire selon l'une quelconque des revendications 1 à 4, dans laquelle l'acide organique est choisi dans le groupe constitué d'acide ascorbique, acide salicylique, acide fumarique, acide benzoïque, acide glutarique, acide lactique, acide citrique, acide malonique, acide acétique, acide glycolique, acide malique, acide adipique, acide succinique, acide aspartique, acide phtalique, acide diacétyltartrique, acide glutamique, acide gluconique, ou leurs mélanges.

6. Composition pour le système respiratoire selon l'une quelconque des revendications 1 à 5, dans laquelle le polymère muco-adhésif est un dérivé de cellulose polymère choisi dans le groupe constitué d'hydroxypropylméthylcelluloses, hydroxypropylcelluloses, polymères de méthylcellulose, polymères de carboxyméthylcellulose, sels de carboxyméthylcellulose, ou leurs mélanges.

7. Composition pour le système respiratoire selon l'une quelconque des revendications 1 à 5, dans laquelle le polymère muco-adhésif est un polymère thermoréversible choisi dans le groupe constitué de poloxamères, éthylhydroxyéthylcelluloses, ou leurs mélanges.

8. Composition pour système respiratoire selon la revendication 6, où la composition a une viscosité allant de 20 mPa.s (cP) à 500 mPa.s (cP).

9. Composition pour le système respiratoire selon la revendication 7, où la composition a une viscosité allant de 10 mPa.s (cP) à 600 mPa.s (cP).

10. Composition pour le système respiratoire selon l'une quelconque des revendications 1 à 9, où la composition comprend en outre un agent d'ajustement du pH choisi dans le groupe constitué de bicarbonate de sodium, phosphate de sodium, hydroxyde de sodium, hydroxyde d'ammonium, stannate de sodium, triéthanolamine, citrate de sodium, succinate disodique, ou leurs mélanges.

11. Composition pour le système respiratoire selon l'une quelconque des revendications 1 à 10, où la composition comprend en outre de 0,01 % à 20 % en poids d'acide pyroglutamique.

12. Composition pour le système respiratoire selon l'une quelconque des revendications 1 à 11, où la composition est une composition nasale.

13. Composition pour le système respiratoire selon la revendication 12, où la composition nasale est choisie dans le groupe constitué de liquides nasaux, pulvérisations nasales, inhalateurs nasaux, poudres nasales, gouttes nasales, ou leurs mélanges.

14. Composition pour le système respiratoire selon la revendication 13, où la composition nasale est une pulvérisation nasale.

15. Composition pour le système respiratoire selon la revendication 14, dans laquelle la pulvérisation nasale comprend de 40 % à 99,98 % en poids d'un véhicule pharmaceutiquement acceptable choisi dans le groupe constitué d'eau, éthanol, propylène glycol, polyéthylène glycol, transcutol, glycérol, un propulseur aérosol liquide, ou leurs mélanges.

16. Utilisation d'une composition selon l'une quelconque des revendications 1 à 11 en tant que médicament.

17. Utilisation d'une composition selon l'une quelconque des revendications 1 à 11 dans la fabrication d'un médicament pour le traitement d'un rhume ou de la grippe.

18. Utilisation selon la revendication 16 ou la revendication 17, dans laquelle le médicament est sous la forme d'une pulvérisation nasale.
